(19) **Europäisches Patentamt / European Patent Office / Office européen des brevets**

(11) **EP 4 549 423 A1**

(12) **EUROPEAN PATENT APPLICATION**
published in accordance with Art. 153(4) EPC

(43) Date of publication:
07.05.2025 Bulletin 2025/19

(21) Application number: 23830915.7

(22) Date of filing: 25.05.2023

(51) International Patent Classification (IPC):
$C07C\ 29/152^{(2006.01)}$ $C07C\ 31/04^{(2006.01)}$
$C07B\ 61/00^{(2006.01)}$

(52) Cooperative Patent Classification (CPC):
(C-Sets available)
C07C 29/1518; C07B 61/00 (Cont.)

(86) International application number:
PCT/JP2023/019530

(87) International publication number:
WO 2024/004464 (04.01.2024 Gazette 2024/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 30.06.2022 JP 2022106619

(71) Applicant: MITSUBISHI GAS CHEMICAL
COMPANY, INC.
Chiyoda-ku
Tokyo 100-8324 (JP)

(72) Inventors:
• HIGASHIDE, Hirofumi
Tokyo 100-8324 (JP)
• KAKIMI, Atsushi
Tokyo 100-8324 (JP)
• AKIYOSHI, Shuusuke
Tokyo 100-8324 (JP)
• ABE, Takanori
Tokyo 100-8324 (JP)

(74) Representative: Vossius & Partner
Patentanwälte Rechtsanwälte mbB
Siebertstrasse 3
81675 München (DE)

(54) **METHANOL PRODUCTION METHOD AND METHANOL PRODUCTION DEVICE**

(57) A method for producing methanol using carbon dioxide and hydrogen as raw materials, the method comprising:

a step (A) of mixing carbon dioxide and hydrogen to obtain a make-up gas;

a step (B) of raising pressure of the make-up gas, and then mixing the make-up gas with a recycled gas recovered from an exit gas of a synthesis reactor to obtain a synthesis reactor-feeding gas;

a step (C) of preheating the synthesis reactor-feeding gas by exchanging heat with the exit gas from the synthesis reactor as a heating source; and

a step (D) of feeding the preheated synthesis reactor-feeding gas to the synthesis reactor and bringing into contact with a catalyst to synthesize methanol.

Figure 1

EP 4 549 423 A1

(52) Cooperative Patent Classification (CPC): (Cont.)

C-Sets
**C07C 29/1518, C07C 31/04**

## Description

Technical Field

The present invention relates to a method for producing methanol and a methanol production apparatus.

Background Art

Industrial production of methanol is performed by reacting, on a catalyst, synthetic raw material gas including carbon monoxide, carbon dioxide, and hydrogen as main components obtained by reforming a fossil fuel as a raw material. In such a process, a large amount of thermal energy is required in the reforming process for producing synthesis raw material gas from raw materials, and thus petrochemical resources are burned and $CO_2$ is emitted.

In recent years, technology for synthesizing methanol from carbon dioxide recovered from exhaust gas, the atmosphere, and the like and hydrogen recovered from water and the like has attracted attention in place of conventional processes using fossil fuels as raw materials. In the methanol synthesis process that uses carbon dioxide and hydrogen as raw materials, there is no reforming process for fossil fuels, and in addition to not emitting $CO_2$, the synthetic raw material gas contains almost no inert gases such as $N_2$ or $CH_4$, and thus the amount of inert gas discharged from the synthetic circulation system is small, and $CO_2$ and the like generated when the exhaust gas is treated can be suppressed.

Patent Literature 1 discloses a carbon dioxide reduction system including a transportation path for separating carbon dioxide from exhaust gas and transporting carbon dioxide, means that heats carbon dioxide, and a reduction apparatus, and describes apparatus for synthesizing methanol as an example of this reduction apparatus.

Patent Literature 2 refers to the synthesis of methanol from carbon dioxide and hydrogen and a downstream distillation process, and describes that the gas passing through a high pressure separator for the synthesis process is partially recycled to upstream of the synthesis process, and that the gas passing through a low pressure separator is fed to the distillation process.

Citation List

Patent Literature

> Patent Literature 1: WO 2019/163968
> Patent Literature 2: Japanese Translation of PCT International Application Publication No. 2019-527691

Summary of Invention

Technical Problem

As described above, a method for producing methanol using carbon dioxide and hydrogen as raw materials is known, but specific conditions for the method for producing methanol have not been mentioned.

In view of the above circumstances, an object of the present invention is to provide a specific method for producing methanol using carbon dioxide and hydrogen as raw materials.

Solution to Problem

As a result of intensive studies to solve the above problems, the present inventors have found that methanol can be efficiently produced from carbon dioxide and hydrogen by a production method including specific steps, and have completed the present invention. That is, the present invention is as follows.

> [1] A method for producing methanol using carbon dioxide and hydrogen as raw materials, the method comprising:
>
>> a step (A) of mixing carbon dioxide and hydrogen to obtain a make-up gas;
>> a step (B) of raising pressure of the make-up gas, and then mixing the make-up gas with a recycled gas recovered from an exit gas of a synthesis reactor to obtain a synthesis reactor-feeding gas;
>> a step (C) of preheating the synthesis reactor-feeding gas by exchanging heat with the exit gas from the synthesis reactor as a heating source; and
>> a step (D) of feeding the preheated synthesis reactor-feeding gas to the synthesis reactor and bringing into contact with a catalyst to synthesize methanol.

[2] The method for producing methanol according to [1], further comprising a step (E) of cooling the exit gas by exchanging heat with the synthesis reactor-feeding gas as a cooling source.

[3] The method for producing methanol according to [2], further comprising a step (F) of performing gas-liquid separation on the exit gas cooled in the step (E) to obtain a gas phase containing an unreacted gas.

[4] The method for producing methanol according to [3], further comprising a step (G) of discharging at least a part of the gas phase obtained in the step (F) as a purge gas out of a synthesis system, recovering a part or all of the remaining gas phase as the recycled gas and mixing with the make-up gas.

[5] The method for producing methanol according to [3], further comprising:

a step (H) of recovering at least a part of the gas phase obtained in the step (F) as a purge gas, and separating the purge gas into a first separated stream and a second separated stream;
a step (I) of discharging a part of the first separated stream out of the synthesis system as an unrecovered gas, recovering a part or all of the remaining first separated stream as a recovered gas and mixing with the make-up gas; and
a step (J) of discharging the second separated stream out of the synthesis system.

[6] The method for producing methanol according to [2] or [3], further comprising:

a step (K) of lowering pressure of a liquid phase obtained by gas-liquid separation of the exit gas cooled in the step (E); and
a step (L) of feeding a flash gas generated during pressure lowering in the step (K) to a scrubber.

[7] The method for producing methanol according to [6], further comprising a step (M) of reusing, as a raw material, a vent gas generated from the scrubber generated in the step (L).

[8] The method for producing methanol according to [7], wherein in the step (M), depending on a pressure of the vent gas, it is selected either the vent gas is joined with the make-up gas or with a back stream of equipment that recovers carbon dioxide from an exhaust gas or atmosphere.

[9] The method for producing methanol according to [6], further comprising a step (N) of joining the vent gas generated from the scrubber generated in the step (L) with the purge gas.

[10] The method for producing methanol according to [1] or [2], wherein a content ratio between carbon dioxide and hydrogen in the make-up gas is hydrogen/carbon dioxide = 2.5 to 4.0 (volume ratio).

[11] The method for producing methanol according to [1] or [2], wherein a circulation ratio, which is a ratio of a molar flow rate of the recycled gas to a molar flow rate of the make-up gas, is 1.0 to 7.0.

[12] A methanol production apparatus for producing methanol using carbon dioxide and hydrogen as raw materials, the apparatus comprising:

a synthesis reactor;
first mixing means that mixes carbon dioxide and hydrogen to obtain a make-up gas;
a compressor that raises pressure of the make-up gas;
second mixing means that mixes the make-up gas raised in pressure with a recycled gas recovered from an exit gas of the synthesis reactor to obtain a synthesis reactor-feeding gas; and
a first heat exchanger that preheats the synthesis reactor-feeding gas by exchanging heat with the exit gas from the synthesis reactor as a heating source,
wherein the preheated synthesis reactor-feeding gas is brought into contact with a catalyst in the synthesis reactor to synthesize methanol.

[13] The methanol production apparatus according to [12], wherein the first heat exchanger cools the exit gas by exchanging heat with the synthesis reactor-feeding gas as a cooling source.

[14] The methanol production apparatus according to [12] or [13], further comprising a high-pressure separator for obtaining a gas phase containing an unreacted gas by gas-liquid separation of the cooled exit gas.

[15] The methanol production apparatus according to [12] or [13], further comprising purge-gas discharging means for discharging at least a part of the gas phase as a purge gas out of the synthesis system,
wherein a part or all of the remaining gas phase after discharging of the purge gas is recovered as the recycled gas and mixed with the make-up gas by the second mixing means.

[16] The methanol production apparatus according to [12] or [13], further comprising:

purge-gas recovering means for recovering at least a part of the gas phase as a purge gas;
separation equipment for separating the purge gas into a first separated stream and a second separated stream;
unrecovered-gas discharging means for discharging a part of the first separated stream out of the synthesis system as an unrecovered gas;
third mixing means for recovering a part or all of the remaining first separated stream as a recovered gas and mixing with the make-up gas; and
second-separated-stream discharging means for discharging the second separated stream out of the synthesis system.

Advantageous Effects of Invention

The method for producing methanol according to the present invention can reduce $CO_2$ emissions compared to conventional methods for producing methanol from raw material gas derived from fossil fuels.

Brief Description of Drawings

[Figure 1] Figure 1 is a schematic view showing an example of a production apparatus used in a method for producing methanol according to the present invention.
[Figure 2] Figure 2 is a schematic view showing another example of the production apparatus used in the method for producing methanol according to the present invention.
[Figure 3] Figure 3 is a schematic view showing another example of the production apparatus used in the method for producing methanol according to the present invention.
[Figure 4] Figure 4 is a schematic view showing another example of the production apparatus used in the method for producing methanol according to the present invention (multi-stage reaction).

Description of Embodiments

Hereinafter, embodiments of the present invention (hereinafter simply referred to as "the present embodiment") will be described with reference to the drawings as necessary, but the present invention is not limited to the following embodiments. Various modifications are possible for the present invention without departing from the gist thereof. In the drawings, the same elements are denoted by the same reference numerals, and overlapping descriptions are omitted. Unless otherwise specified, positional relationships such as up, down, left, and right are based on the positional relationships shown in the drawings. The dimensional ratios of the drawings are not limited to the illustrated ratios.
The method for producing methanol according to the present embodiment is a method for producing methanol using carbon dioxide and hydrogen as raw materials, the method including:

a step (A) of mixing carbon dioxide and hydrogen to obtain a make-up gas;
a step (B) of raising pressure of the make-up gas, and then mixing the make-up gas with a recycled gas recovered from an exit gas of a synthesis reactor to obtain a synthesis reactor-feeding gas;
a step (C) of preheating the synthesis reactor-feeding gas by exchanging heat with the exit gas from the synthesis reactor as a heating source; and
a step (D) of feeding the preheated synthesis reactor-feeding gas to the synthesis reactor and bringing into contact with a catalyst to synthesize methanol.

<Method for producing methanol>

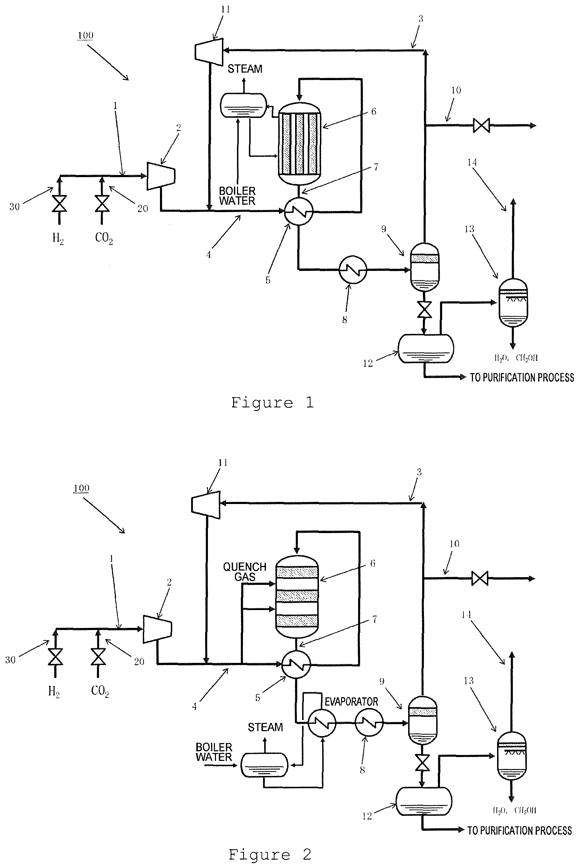

The method for producing methanol according to the present embodiment will be described below using a methanol production apparatus of Figures 1 to 3. The method for producing methanol according to the present embodiment is not limited to the embodiments using the production apparatus shown in Figures 1 to 3.

[Step (A)]

A step (A) in the method for producing methanol according to the present embodiment is a step of mixing carbon dioxide and hydrogen to obtain a make-up gas 1. In this step, carbon dioxide and hydrogen introduced from a carbon dioxide introduction pipe 20 and a hydrogen introduction pipe 30 respectively are mixed within a line 1.
The content ratio between carbon dioxide and hydrogen in the make-up gas is preferably hydrogen/carbon dioxide = 2.5 to 4.0 (volume ratio), more preferably 2.8 to 3.7, and further preferably 2.8 to 3.5. When the content ratio between carbon dioxide and hydrogen in the make-up gas is 2.5 or more, the reaction proportion of carbon dioxide tends to increase and the amount of by-products tends to decrease. When this content ratio is 4.0 or less, the hydrogen reaction proportion and production efficiency tend to increase.

The content ratio between carbon dioxide and hydrogen in the make-up gas can be adjusted to an appropriate proportion by controlling the flow rate of each raw material from the carbon dioxide introduction pipe and the hydrogen introduction pipe.

Carbon dioxide and hydrogen used as raw materials are not particularly limited, but from the viewpoint of reducing $CO_2$ emissions, it is preferable to use carbon dioxide recovered from exhaust gases and the atmosphere, and hydrogen obtained using renewable energy. More specifically, for carbon dioxide, for example, there can be used carbon dioxide derived from thermal power generation, derived from biomass power generation, derived from natural gas power generation, derived from by-products during chemical substance manufacturing, derived from accompanying digestive gas from sewage treatment, derived from methane fermentation, derived from mineral processes, derived from the steel industry, and derived from waste incinerator. For hydrogen, for example, there can be used by-product hydrogen from petroleum refining facilities, by-product hydrogen from chemical processes, blue hydrogen such as by-product hydrogen using CCS in combination, hydrogen whose gas composition is adjusted by PSA or the like, water electrolysis hydrogen, salt solution electrolysis hydrogen, hydrogen obtained by other electrolysis technology, and hydrogen obtained by steam reforming.

From the viewpoint of production efficiency, the purity of carbon dioxide used as a raw material is preferably 98% or more, preferably 99% or more, and more preferably 99.9% or more.

From the viewpoint of production efficiency, the purity of hydrogen used as a raw material is preferably 99% or more, preferably 99.9% or more, and more preferably 99.99% or more.

[Step (B)]

A step (B) in the method for producing methanol according to the present embodiment is a step of raising pressure of the make-up gas 1, and then mixing the make-up gas 1 with a recycled gas 3 recovered from an exit gas of a synthesis reactor 6 to obtain a synthesis reactor-feeding gas 4. A compressor 2, for example, is used to raise pressure of the make-up gas.

The recycled gas 3 is a gas containing an unreacted component of the synthesis reactor-feeding gas 4 used in the methanol synthesis reaction, and is recovered from an exit gas 7 of the synthesis reactor 6.

The circulation ratio, which is the ratio of the molar flow rate of the recycled gas 3 to the molar amount of the make-up gas 1, is preferably 1.0 to 7.0, more preferably 3.0 to 7.0, and further preferably 4.0 to 6.0. When the circulation ratio is 1.0 or more, the amount of by-products tend to decrease, and when the circulation ratio is 7.0 or less, the production efficiency tends to increase.

The above circulation ratio can be measured from the ratio between the flow rate of the recycled gas 3 and the flow rate of the raw material gas 1 (make-up gas).

[Step (C)]

The step (C) in the method for producing methanol according to the present embodiment is a step of preheating the synthesis reactor-feeding gas 4 by exchanging heat with the exit gas 7 from the synthesis reactor 6 as a heating source. A mutual heat exchanger 5 is used for heat exchange, for example.

[Step (D)]

A step (D) in the method for producing methanol according to the present embodiment is a step of feeding the preheated synthesis reactor-feeding gas 4 to the synthesis reactor 6 and bringing into contact with a catalyst to synthesize methanol.

The gas temperature at the entrance of the synthesis reactor is appropriately set depending on the type and amount of the catalyst, the shape of the reactor, the reaction pressure, and the like, but is preferably 170 to 260°C, more preferably 170 to 220°C, and further preferably 170 to 200°C. When the entrance gas temperature is 170°C or more, the reactivity tends to improve, and when the entrance gas temperature is 260°C or less, the equipment cost tends to be reduced.

The gas pressure at the entrance of the synthesis reactor is preferably 4.9 to 14.7 MPaG, more preferably 5.0 to 11.0 MPaG, and further preferably 5.0 to 10.0 MPaG. When the entrance gas pressure is 4.9 MPaG or more, the reactivity tends to improve, and when the entrance gas pressure is 14.7 MPaG or less, the production efficiency tends to increase.

The synthesis reactor-feeding gas 4 that is fed to the synthesis reactor has a relation (M) of mol% of CO, $CO_2$, and

$H_2$ calculated by the following formula:

$$M = (H_2 \text{ mol\%}) / (2 \times CO \text{ mol\%} + 3 \times CO_2 \text{ mol\%})$$

wherein M is preferably 1.3 to 5.0, more preferably 1.5 to 4.0. When the M value is 1.3 or more, the amount of by-products tend to decrease, and when the M value is 5.0 or less, the production efficiency tends to increase.

From the viewpoint of maintaining reactivity, suppressing by-products, and protecting the catalyst, the reaction temperature in the synthesis reactor 6 is preferably 200 to 300°C, more preferably 200 to 280°C, and further preferably 200 to 270°C.

The synthesis reactor 6 is not particularly limited, and preferably has a mechanism (heat removal mechanism) for removing heat generated by the reaction from the catalyst layer. Specific examples thereof include a heat-exchange-type tubular reactor having the shell side serving as an evaporator, and an adiabatic type reactor. Herein, Figure 1 shows an example using a heat-exchange-type tubular reactor as a synthesis reactor, and Figure 2 shows an example using an adiabatic reactor as a synthesis reactor.

When using a heat-exchange-type tubular reactor, the reaction temperature is controlled by indirect heat exchange with pressurized boiling water to obtain saturated vapor (steam). Boiling water is circulated through a steam drum and the shell side of the tubular reactor to recover steam from the steam drum. The steam obtained in this synthesis system is preferably used as a heating source for the purification process of the methanol solution on downstream of the synthesis process. The pressurized boiling water preferably has a temperature of 220°C to 260°C.

When adopting the adiabatic type reactor, it has one or more catalyst layers inside. In the case of two layers or more, a part of the synthesis reactor-feeding gas 4 is branched as a cooling gas for an intermediate layer and fed as a quench gas to control the reaction temperature, and an evaporator is provided as heat recovery equipment for the reactor exit gas 7 to recover steam, which may also be used as a heating source for the purification process of the methanol solution on the downstream.

The catalyst used for the synthesis is preferably a methanol synthesis catalyst containing copper atoms and zinc atoms as essential components. Such a catalyst is reduced from an oxide state by a reducing gas such as hydrogen, carbon monoxide, or a mixed gas thereof, thereby activating copper and having catalytic activity. The catalyst may contain aluminum atoms and/or chromium atoms as a main third component in addition to copper atoms and zinc atoms. A catalyst containing copper and zinc as essential components can be prepared by a known method. Such a catalyst can be prepared, for example, by the methods described in Japanese Patent Publication No. 51-44715, Japanese Patent No. 2695663, Japanese Patent Publication No. 6-35401, Japanese Patent Application Laid-Open No. 10-272361, and Japanese Patent Application Laid-Open No. 2001-205089.

A preferable catalyst is a methanol synthesis catalyst containing copper atoms and zinc atoms in an atomic ratio (copper/zinc) of 2.0 to 3.0 and containing aluminum atoms. Examples of such a catalyst include the catalyst prepared by the method described in Japanese Patent Application Laid-Open No. 8-299796 and the catalyst described in International Publication No. WO2011/048976.

Specific examples of a preferable catalyst include the catalysts used in Examples and Comparative Examples, such as Examples 2 and 3, of International Publication No. WO2011/048976. A more preferable atomic ratio of copper atoms and zinc atoms (copper/zinc) in the catalyst is in the range of 2.1 to 3.0. In addition, a methanol synthesis catalyst containing 3 to 20% by mass of alumina is more preferable. Such a catalyst can be prepared, for example, by the method described in International Publication No. WO2011/048976, as described above. More specifically, for example, this catalyst is prepared by a production method comprising: a step of mixing an aqueous solution containing copper, an aqueous solution containing zinc, and an aqueous alkali solution to form a precipitate containing copper and zinc; a step of mixing the obtained precipitate with an alumina hydrate having a pseudo-boehmite structure to obtain a mixture; and a step of molding the obtained mixture so that the density becomes 2.0 to 3.0 g/mL. Herein, examples of the molding method include tableting, extrusion molding, and rolling granulation. The catalyst used in the present embodiment is not limited to the above catalyst and the catalyst prepared by the above preparation method, and may be another catalyst having equivalent methanol synthesis activity.

[Step (E)]

A step (E) in the method for producing methanol according to the present embodiment is a step of cooling the exit gas 7 of the synthesis reactor by exchanging heat with the synthesis reactor-feeding gas 4 as a cooling source. For heat exchange, for example, the mutual heat exchanger 5 used in the step (C) is used.

The exit gas from the synthesis reactor 6 may be cooled through heat exchange using the synthesis reactor-feeding gas 4 as a cooling source, and then further cooled through a cooler 8.

[Step (F)]

A step (F) in the method for producing methanol according to the present embodiment is a step of obtaining a gas phase containing an unreacted gas by gas-liquid separation of the exit gas cooled in the step (E). Gas-liquid separation of the cooled exit gas is performed by using a high-pressure separator 9, for example.

[Step (G)]

A step (G) in the method for producing methanol according to the present embodiment is a step of discharging at least a part of the gas phase obtained in the step (F) out of the synthesis system as a purge gas 10, recovering a part of the remaining gas phase as the recycled gas 3, and mixing with the make-up gas 1.

At least a part of the gas phase containing the unreacted gas gas-liquid separated in the high-pressure separator 9 in the step (F) is discharged out of the synthesis system through a purge gas discharge pipe 10. Specifically, the inert gas and impurities contained in the raw materials and by-products (hydrocarbons, organic gas impurities such as methyl formate) produced by the synthesis reaction are discharged out of the system as a purge gas. In the method for producing methanol according to the present embodiment, the inert gas in the make-up gas is less than in the conventional process, and the amount of the inert gas discharged by the purge gas is also less. Therefore, it is possible to operate with less purge gas than in the conventional process.

Meanwhile, a part or all of the remaining gas phase obtained in the step (F) is recovered as the recycled gas 3 and mixed with the make-up gas 1 after being raised in pressure. The recycled gas 3 may be compressed by a compressor 11 or the like before being mixed with the make-up gas 1.

The purge gas 10 is a combustible gas containing $CH_4$, CO, $CO_2$, $H_2$, and the like as main components, and thus is often combusted as in the conventional process, and the carbon contained in the purge gas is often discharged as $CO_2$.

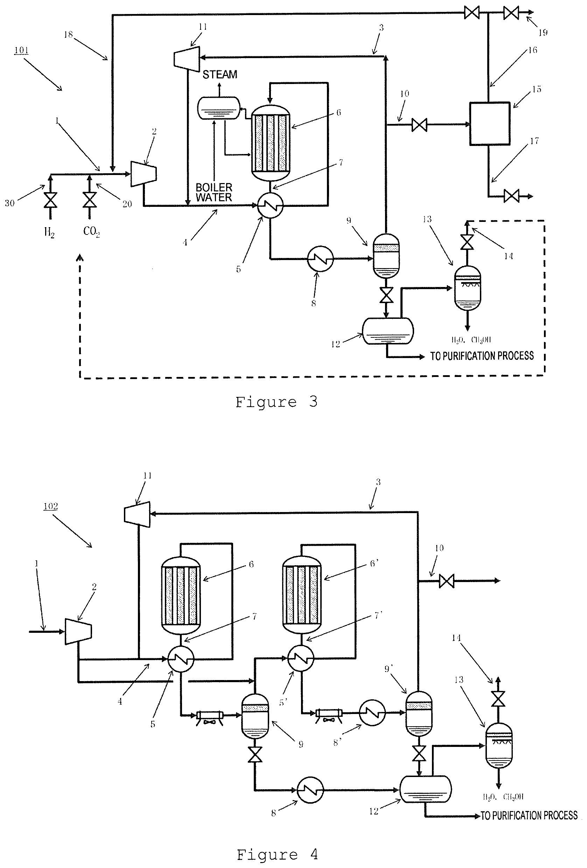

The method for producing methanol according to the present embodiment may further include the following steps (H) to (J). Herein, the apparatus shown in Figure 3 is an example of an apparatus used when performing the method including steps (H) to (J) of the present embodiment.

[Step (H)]

A step of recovering at least a part of the gas phase obtained in the step (F) as the purge gas 10 and separating the purge gas into a first separated stream 16 and a second separated stream 17

[Step (I)]

A step of discharging a part of the first separated stream 16 out of the synthesis system as an unrecovered gas 19, recovering a part or all of the remaining stream as a recovered gas 18, and mixing with the make-up gas 1

[Step (J)]

A step of discharging the second separated stream 17 out of the synthesis system

In the apparatus of Figure 3, the purge gas 10 passes through separation equipment 15 and is separated into the first separated stream 16 and the second separated stream 17. Examples of the separation equipment 15 include devices that remove impurities from purge gas, such as demisters, adsorbents, scrubbers, cyclones, vanes, and filters, and devices that separate $H_2$ from purge gas, such as PSA and membrane separators. Impurities to be removed include organic substances such as hydrocarbons and esters, inorganic substances such as chlorine, and solid substances such as iron rust.

When an impurity remover is used in the separation equipment 15, the first separated stream 16 becomes the purified purge gas from which the impurities are removed from the purge gas 10, and the second separated stream 17 becomes the impurity stream. The amount of impurities accumulated in the synthesis system is reduced by the impurity remover, and thus the amount of gas discharged and the $CO_2$ emitted out of the synthesis system can be likely to be reduced.

When the purge gas 10 is cleaned by a scrubber provided in the separation equipment 15, the second separated stream 17 discharged from the separation equipment after cleaning becomes a mixed solution of methanol, water, impurity streams, and the like, and thus is preferably recovered and transferred to a purification process to recover methanol.

When PSA and the membrane separator are used for the separation equipment 15, the first separated stream 16 becomes a gas with a higher $H_2$ concentration than the purge gas 10 and the second separated stream 17 becomes a gas with a higher $CO_2$ concentration than the purge gas 10. All of the first separated stream 16 having a high $H_2$ concentration may be mixed with the make-up gas 1 as the recovered gas 18, a part the stream 16 may be discharged out of the synthesis system as the unrecovered gas 19, or all of the stream 16 may be discharged out of the synthesis system as the unrecovered gas 19. The unrecovered gas with a high $H_2$ concentration can be used as a clean fuel that does not emit $CO_2$. Whereas, the second separated stream 17 is discharged out of the synthesis system. The $CO_2$ contained in the second separated stream 17 may be reused as a raw material, or may be buried in the ground using CCS or the like to reduce $CO_2$ emissions.

The method for producing methanol according to the present embodiment may further include the following steps (K) and (L).

[Step (K)]

A step of lowering pressure of the liquid phase obtained by gas-liquid separation of the exit gas cooled in the step (E)

[Step (L)]

A step of feeding a flash gas generated during pressure lowering in the step (K) to the scrubber

The pressure of liquid phase obtained by gas-liquid separation of the cooled exit gas in the step (E) is lowered by using a low-pressure separator 12 or the like, and then the flash gas generated during pressure lowering in the low-pressure separator 12 is fed to a scrubber 13, and is brought into contact with water to recover methanol and water.

The method for producing methanol according to the present embodiment may further include the following steps (M) and (N).

[Step (M)]

A step of reusing a vent gas generated from the scrubber generated in the step (L) as a raw material

[Step (N)]

A step of joining the vent gas generated from the scrubber generated in the step (L) with the purge gas

The main components of a vent gas 14 generated from the scrubber in the step (L) are $CO_2$ and $H_2$, and therefore may be reused as raw materials (step (M)), or the vent gas 14 may be joined with the purge gas 10 (Step (N)).

In the step (M), depending on the pressure of the vent gas 14, it may be selected either the vent gas 14 is joined with the make-up gas 1, or is joined with a back stream of equipment (not shown) that recovers carbon dioxide from the exhaust gas or the atmosphere.

For example, when the pressure of the vent gas 14 is higher than the pressure of the make-up gas 1 fed to the compressor 2, the vent gas 14 may be joined with the make-up gas 1 and reused. Whereas, when the pressure of the vent gas 14 is lower than the pressure of the make-up gas 1 fed to the compressor 2, the vent gas 14 is joined with $CO_2$ on the back stream of the equipment that recovers carbon dioxide operated at atmospheric pressure or low pressure, and the joined gas is raised in pressure and then may be reused through the carbon dioxide introduction pipe 20. Herein, low pressure refers to, for example, less than 1.0 MPaG.

The aqueous solution containing methanol separated by the low-pressure separator 12 may be fed to a purification process such as distillation. The low-pressure separator 12 and the scrubber 13 are shown as separate devices in Figure 1, but a device obtained by integrating the low-pressure separator 12 and the scrubber 13 may be used.

In the method for producing methanol according to the present embodiment, the synthesis yield decreases and the purge gas flow rate increases as the catalyst activity deteriorates over time. Accordingly $CO_2$ emissions will increase, and it is thus preferable that synthesis pressure, reaction temperature, circulation ratio (recycled gas flow rate / make-up gas flow rate), raw material composition (hydrogen flow rate / carbon dioxide flow rate), any one of these elements, or two or more elements are adjusted appropriately to keep the synthesis yield constant and continue to operate with minimal $CO_2$ emissions.

<Methanol production apparatus>

The methanol production apparatus of the present embodiment is an apparatus for performing the above method for producing methanol, and examples thereof include the apparatuses shown in the schematic views of Figures 1 to 3.

A methanol production apparatus 100 of the present embodiment is a methanol production apparatus for producing methanol using carbon dioxide and hydrogen as raw materials, the apparatus comprising:

a synthesis reactor 6;

first mixing means (join portion of a carbon dioxide introduction pipe 20 and a hydrogen introduction pipe 30) that mixes carbon dioxide and hydrogen to obtain a make-up gas;

a compressor 2 that raises pressure of the make-up gas;

second mixing means (join portion of a lines 1 and 3) that mixes the make-up gas raised in pressure with a recycled gas recovered from an exit gas of the synthesis reactor to obtain a synthesis reactor-feeding gas; and

a first heat exchanger 5 that preheats the synthesis reactor-feeding gas by exchanging heat with the exit gas from the synthesis reactor as a heating source,

wherein the preheated synthesis reactor-feeding gas is brought into contact with a catalyst in the synthesis reactor 6 to synthesize methanol.

In the methanol production apparatus according to the present embodiment, the first heat exchanger 5 may cool the exit gas from the synthesis reactor 6 by exchanging heat with the synthesis reactor-feeding gas as a cooling source.

The methanol production apparatus according to the present embodiment may further include a high-pressure separator 9 for obtaining a gas phase containing unreacted gas by performing gas-liquid separation of the exit gas cooled by the first heat exchanger 5.

The methanol production apparatus according to the present embodiment further comprises purge-gas discharging means (line 10) for discharging at least a part of the gas phase obtained in the high-pressure separator 9 out of the synthesis system as a purge gas,

wherein a part or all of the remaining gas phase after discharging of the purge gas may be recovered as the recycled gas and mixed with the make-up gas by the second mixing means (join portion of lines 1 and 3).

An apparatus 101 for producing methanol according to the present embodiment may further comprise:

purge-gas discharging means (line 10) for recovering at least a part of the gas phase obtained in the high-pressure separator 9 as a purge gas;

separation equipment 15 for separating the purge gas into a first separated stream and a second separated stream;

unrecovered-gas discharging means (line 19) for discharging a part of the first separated stream out of the synthesis system as an unrecovered gas;

third mixing means (join portion of the lines 1 and 18) for recovering a part or all of the remaining first separated stream as a recovered gas and mixing with the make-up gas; and

second-separated-stream discharging means (line 17) for discharging the second separated stream out of the synthesis system.

<Multi-stage reaction>

The method and methanol production apparatus according to the present embodiment also include a production method and production apparatus 102 by multi-stage reaction using a device in which a first synthesis reactor 6 and a second synthesis reactor 6' are arranged in series as shown in Figure 4. The multi-stage reaction is performed in the same manner as the methanol production method using one synthesis reactor described above, except that the exit gas from the first synthesis reactor 6 is cooled by the heat exchanger 5 and is gas-liquid separated in the separator 9, and then is preheated in a heat exchanger 5' and fed again to the second synthesis reactor 6' different from the first synthesis reactor. Figure 4 does not show separation equipment for separating the purge gas, but separation equipment for separating the purge gas may be provided in the multi-stage reaction as in the above-described methanol production method.

Example 1

Hereinafter, the method and methanol production apparatus according to the present invention will be described in detail with reference to Examples and Comparative Examples, but the present invention is not limited thereto.

The catalyst used for methanol synthesis was any of a catalyst prepared by the method described in Example 1 of Japanese Patent Publication No. 51-44715 (methanol synthesis catalyst A); a catalyst prepared by the method described in Example 1 of Japanese Patent Laid-Open No. 8-299796 (methanol synthesis catalyst B); a catalyst prepared by the method described in Example 3 of International Publication No. WO 2011/048976 (methanol synthesis catalyst C); or a catalyst prepared by the method described in Comparative Example 4 of Japanese Patent Laid-Open No. 8-299796 (methanol synthesis catalyst D).

[Example 1]

In Example 1, the production apparatus shown in Figure 1 was used. Each condition was as follows. By using $CO_2$ and $H_2$ as raw material gases and adjusting the flow rates of $CO_2$ and $H_2$, the composition of the make-up gas 1 was

adjusted to $H_2/CO_2 = 3.0$, and methanol was synthesized under the conditions of a reactor entrance pressure of 8.0 MPaG and a circulation ratio of 2.7. The methanol synthesis catalyst B was used in the synthesis reactor 6. The pressure of the make-up gas 1 was raised to 8.07 MPaG by the main compressor 2. The make-up gas 1 raised in pressure was joined with the recycled gas 3 to be become the synthesis reactor-feeding gas 4, and was heat-exchanged with the exit gas 7 of the synthesis reactor flowing through the exit of the synthesis reactor 6, thereby preheating the gas to be fed to the synthesis reactor to 200°C. A heat-exchange type tubular reactor having an inner tube made of stainless steel was used as the synthesis reactor 6. The fluid pressure in the catalyst layer was 8.00 to 8.02 MPaG and the temperature was between 200°C and 234°C. This temperature range was significantly preferable as the temperature at which the catalyst was used. In this case, the temperature of the pressurized boiling water as the coolant was 227°C.

The exit gas 7 from the synthesis reactor 6 was cooled by the heat exchanger 5 and the cooler 8 to 45°C, which is below the dew point of methanol, to promote condensation of methanol. The exit gas 7 of the reactor flowing through the exit of the reactor was cooled by the synthesis reactor-feeding gas 4. The molar flow rate of the recycled gas 3 generated from the high-pressure separator 9 was controlled to be 2.7 times the molar flow rate of the make-up gas 1, and as a result, the molar flow ratio of the purge gas 10 to the unreacted gas flow before separation of the purge gas 10 was 1.1%.

Synthetic carbon yield in Example 1 is represented as a ratio of methanol molar flow rate in crude methanol to $CO_2$ molar flow rate in the make-up gas 1, and was 95.5%.

[Comparative Example 1]

In Comparative Example 1 as well, the production apparatus shown in Figure 1 was used in the same manner as in Example 1. The difference from Example 1 was the composition of the make-up gas 1 as a raw material. Assuming a steam-reformed gas obtained by subjecting natural gas to a steam-reforming reaction, which is widely used as a raw material for methanol synthesis, a representative composition thereof was used as the composition of the make-up gas 1. The sum of the CO molar flow rate and the $CO_2$ molar flow rate of the make-up gas 1, that is, the carbon raw material contributing to the production of methanol was set to be all the same in Example 1, Comparative Example 1, and Comparative Example 2. By adjusting the amount of the catalyst introduced into the synthesis reactor 6, the main operating conditions such as the synthesis reactor entrance pressure, circulation ratio, and pressurized boiling water temperature were set to predetermined values, and the synthetic carbon yield, that is, the amount of methanol produced, was set to be all the same in Example 1, Comparative Example 1, and Comparative Example 2.

The exit gas 7 from the synthesis reactor 6 was cooled by the heat exchanger 5 and the cooler 8 to 45°C, which is below the dew point of methanol, to promote condensation of methanol. The exit gas 7 of the reactor flowing through the exit of the reactor was cooled by the synthesis reactor-feeding gas 4. The molar flow rate of the recycled gas 3 generated from the high-pressure separator 9 was controlled to be 2.7 times the molar flow rate of the make-up gas 1, and as a result, the molar flow ratio of the purge gas to the unreacted gas flow before separation of the purge gas 10 was 9.3%.

Synthetic carbon yield in Comparative Example 1 is represented as a ratio of methanol molar flow rate in crude methanol to sum of CO molar flow rate and $CO_2$ molar flow rate in the make-up gas 1, the synthetic carbon yield was set to 95.5%, which is the same as in Example 1. In this case, the amount of catalyst was adjusted to about 1.1 times that of Example 1.

The difference between Example 1 and Comparative Example 1 was the composition of the make-up gas 1. In Example 1, almost no inert gas existed in the make-up gas 1, but in Comparative Example 1, inert gas typified by $CH_4$ existed. $CH_4$ that does not contribute to methanol production accumulated in the synthesis system and was discharged as a purge gas. As a result, the $CO_2$ emission during the combustion process of the purge gas and vent gas was 5.5 times that of Example 1.

[Comparative Example 2]

In Comparative Example 2 as well, the production apparatus shown in Figure 1 was used in the same manner as in Example 1. The difference from Example 1 was the composition of the make-up gas 1 as a raw material. Assuming a two-stage reformed gas, which is widely used as a raw material for current methanol synthesis, obtained by subjecting natural gas to a steam-reforming reaction followed by partial oxidation reaction, a representative composition thereof was used as the composition of the make-up gas 1.

The exit gas from the synthesis reactor 6 was cooled by the heat exchanger 5 and the cooler 8 to 45°C, which is below the dew point of methanol, to promote condensation of methanol. The exit gas 7 of the reactor flowing through the exit of the reactor was cooled by the synthesis reactor-feeding gas 4. The molar flow rate of the recycled gas 3 generated from the high-pressure separator 9 was controlled to be 2.7 times the molar flow rate of the make-up gas 1, and as a result, the molar flow ratio of the purge gas to the unreacted gas flow before separation of the purge gas 10 was 2.9%.

Synthetic carbon yield in Comparative Example 2 is represented as a ratio of methanol molar flow rate in crude methanol to sum of CO molar flow rate and $CO_2$ molar flow rate in the make-up gas 1, the synthetic carbon yield was set to 95.5%, which is the same as in Example 1. In this case, the amount of catalyst was adjusted to about 0.6 times that of Example 1.

The difference between Example 1 and Comparative Example 2 was the composition of the make-up gas 1. The $CO_2$ emission during the combustion process of the purge gas and vent gas was 2.3 times that of Example 1.

Table 1 shows various conditions and $CO_2$ emission ratios in Example 1 and Comparative Examples 1 and 2.

[Table 1]

| | | | Comparative Example 1 | Comparative Example 2 | Example 1 |
|---|---|---|---|---|---|
| Type of raw material gas | | | Steam reformed gas | Two-stage reformed gas | CO2, H2 |
| Reactor entrance pressure | | MPaG | 8.0 | 8.0 | 8.0 |
| Circulation ratio *1 | | - | 2.7 | 2.7 | 2.7 |
| Temperature of pressurized boiling water | | °C | 227 | 227 | 227 |
| Synthetic carbon yield *2 | | % | 95.5 | 95.5 | 95.5 |
| Composition of raw material gas | CO2 | mol% | 8 | 8 | 25 |
| | CO | mol% | 15 | 21 | 0 |
| | H2 | mol% | 73 | 70 | 75 |
| | Inert | mol% | 4 | 2 | 0 |
| CO2 emission ratio during combustion of purge gas and vent gas based on Comparative Example 1 *3 | | - | 1.00 | 0.43 | 0.18 |
| *1 Circulation ratio = molar flow rate of recycled gas / molar flow rate of make-up gas<br>*2 Synthetic carbon yield = molar flow rate of methanol product / sum of molar flow rates of CO and CO2 in make-up gas (percentage)<br>*3 Molar flow rate of CO2 emission during combustion = CO2 molar flow rate + CO molar flow rate + CH4 molar flow rate + CH3OH molar flow rate in discharged gas | | | | | |

[Example 2]

Example 2 was the same process as in Example 1 and was used for comparison with Examples 3 to 6. In Example 2, the production apparatus shown in Figure 1 was used. Each condition was as follows. By using $CO_2$ and $H_2$ as raw material gases and adjusting the flow rates of $CO_2$ and $H_2$, the composition of the make-up gas 1 was adjusted to $H_2/CO_2$ = 3.0, and the composition and flow rate were set to be the same as in the make-up gas 1 in Example 1. Methanol synthesis was performed under conditions of a reactor entrance pressure of 8.0 MPaG and a circulation ratio of 3.0. The methanol synthesis catalyst B was used in the synthesis reactor 6, and the catalytic activity was 0.8 times that of Example 1. The pressure was raised to 8.07 MPaG by the main compressor 2. The make-up gas 1 raised in pressure was joined with the recycled gas 3 to be become the synthesis reactor-feeding gas 4, and was heat-exchanged with the exit gas 7 of the synthesis reactor flowing through the exit of the synthesis reactor 6, thereby preheating the gas to be fed to the synthesis reactor to 200°C. A heat-exchange type tubular reactor having an inner tube made of stainless steel was used as the synthesis reactor 6. The fluid pressure in the catalyst layer was 8.00 to 8.02 MPaG and the temperature was between 200°C and 233°C. The temperature of the pressurized boiling water as the coolant was 227°C.

The exit gas 7 from the synthesis reactor 6 was cooled by the heat exchanger 5 and the cooler 8 to 45°C, which is below the dew point of methanol, to promote condensation of methanol. The exit gas 7 of the reactor flowing through the exit of the reactor was cooled by the synthesis reactor-feeding gas 4. The molar flow rate of the recycled gas 3 generated from the high-pressure separator 9 was controlled to be 3.0 times of the molar flow rate of the make-up gas 1, and as a result, the molar flow ratio of the purge gas to the unreacted gas flow before separation of the purge gas 10 was 1.5%.

Synthetic carbon yield in Example 2 is represented as a ratio of methanol molar flow rate in crude methanol to $CO_2$ molar flow rate in the make-up gas 1, the synthetic carbon yield was 93.8%. Synthetic hydrogen yield is represented by the sum of twice the molar flow rate of methanol in crude methanol and the molar flow rate of water to the molar flow rate of $H_2$ in the make-up gas 1, and was 94.3%.

[Example 3]

In Example 3, the production apparatus shown in Figure 3 was used. By providing the separation equipment 15 that separates the purge gas 10 of Example 2, the synthetic carbon yield was improved as compared with that of Example 2. In Example 3, a scrubber was provided as the separation equipment. By sprinkling water with the scrubber, the methanol present in the purge gas 10 was recovered together with water in the second separated stream 17 and fed to the refining process to become a product. The remaining gas became the first separated stream 16 and was discharged out of the system as the unrecovered gas 19.

Synthetic carbon yield in Example 3 is represented as a ratio of methanol molar flow rate in crude methanol to $CO_2$ molar flow rate in the make-up gas 1, the synthetic carbon yield was 94.1%. The yield was improved by 0.3% as compared with Example 2.

[Example 4]

In Example 4 as well, the production apparatus shown in Figure 3 was used. By providing a PSA-$H_2$ device downstream of the scrubber of the separation equipment 15 of Example 3, the synthetic hydrogen yield was improved as compared with Example 1. The $H_2$ present in the purge gas 10 passing through the PSA was separated into a first separated stream 16 with a recovery rate of 80% and a purity of 100% after recovery. The second separated stream 17 contained remaining gas of the purge gas 10 having a carbon concentration higher than that of the purge gas 10 in addition to scrubber wastewater.

$H_2$ is expensive, and reusing it as a recovered gas 18 for a raw material of methanol synthesis can provide an economic advantage. $H_2$ has a high utility value as a clean fuel that does not generate $CO_2$, and it is beneficial to feed it out of the system as an unrecovered gas 19. In Example 4, the total amount of the first separated stream 16 was recovered as the recovered gas 18, and the flow rate of $H_2$ fed as the raw material was reduced accordingly.

Compared with Examples 2 and 3, in Example 4, the amount of $H_2$ fed as a raw material was reduced by about 3%. Synthetic hydrogen yield is represented by the sum of twice the molar flow rate of methanol in crude methanol and the molar flow rate of water to the molar flow rate of $H_2$ in the make-up gas 1, and was 98.4%. Compared with Examples 2 and 3, improvement by about 4% was obtained.

Table 2 shows various conditions and $CO_2$ emission ratios in Examples 2 to 4.

[Table 2]

| | | Example 2 | Example 3 | Example 4 |
|---|---|---|---|---|
| | | No separation equipment | Scrubber provided | PSA equipment provided |
| Flow rate of raw material gas | kmol/h | 269.6 | 269.6 | 261.3 |
| CO2 | kmol/h | 67.3 | 67.3 | 67.3 |
| H2 | kmol/h | 202.1 | 202.1 | 193.8 |
| Synthesis pressure | MPaG | 8.0 | ← | ← |
| Temperature of pressurized boiling water | °C | 227 | ← | ← |
| Flow rate of recycled gas | kmol/h | 808.8 | ← | ← |
| Synthetic carbon yield[*1] | % | 93.8 | 94.1 | 94.1 |
| Synthetic hydrogen yield [*2] | % | 94.3 | ← | 98.4 |
| CO2 emission ratio during combustion of purge gas and vent gas based on Example 3-1[*3] | - | 1.0 | ← | ← |
| *1 Synthetic carbon yield = molar flow rate of methanol product / molar flow rate of $CO_2$ in make-up gas (percentage) *2 Synthetic hydrogen yield = sum of molar flow rates of methanol product and H2 converted to water / molar flow rate of H2 in make-up gas (percentage) *3 Molar flow rate of $CO_2$ emission during combustion = CO2 molar flow rate + CO molar flow rate + CH4 molar flow rate + CH3OH molar flow rate in discharged gas | | | | |

[Example 5]

In Example 5, the production apparatus shown in Figure 3 was used. By providing a device for removing impurities from the purge gas 10, such as a demister, an adsorbent, a scrubber, a cyclone, a vane, or a filter, as the separation equipment 15 that separates the purge gas 10 of Example 2, organic substances such as hydrocarbons and esters present in the system, inorganic substances such as chlorine, and solid impurities such as iron rust can be discharged out of the system and can be prevented from accumulating in the system. A purge gas from which impurities had been removed was present in the first separated stream 16, a part or all of the purge gas was recovered as the recovered gas 18 for a raw material, and a part of the remaining purge gas was fed out of the synthesis system as the unrecovered gas 19. Impurities were present in the second separated stream 17 and discharged out of the synthesis system.

In Examples 1 and 2, when the carbon yield is increased and the amount of the purge gas 10 is reduced, impurities accumulate in the system. In particular, in hydrocarbon-based organic gases, accumulation in the system is suppressed by maintaining the purge gas 10 molar flow rate at 1% or more, more preferably 2% or more relative to the make-up gas 1 molar flow rate. The accumulation of these impurities adversely affects the catalytic activity and synthesis reaction performance, and therefore it is preferable to maintain a constant molar flow rate of the purge gas 10, thus limiting the upper limits of the synthetic carbon yield and synthetic hydrogen yield.

In Example 5, impurities are removed by the separation equipment 15, and thus even when the recovered gas 18 is joined with the make-up gas 1, the impurities will not be accumulated in the synthesis system. Therefore, it is possible to reduce the molar flow rate of the purge gas 10 as compared with Examples 1 and 2, and the upper limits of the synthetic carbon yield and the synthetic hydrogen yield are not limited. In addition, it is possible to reduce the $CO_2$ emission when the gas discharged out of the synthesis system is combusted.

[Example 6]

In Example 6 as well, the production apparatus shown in Figure 3 was used. The main components of the vent gas 14 are $H_2$ and $CO_2$, and thus are reused as raw material gases. Accordingly, the amounts of $H_2$ and $CO_2$ fed to the raw material are reduced, and the carbon yield and hydrogen yield are improved. In addition, the $CO_2$ emission when the gas discharged out of the synthesis system is combusted is reduced.

Industrial Applicability

The present invention has industrial applicability in the method and methanol production apparatus.

**Claims**

1. A method for producing methanol using carbon dioxide and hydrogen as raw materials, the method comprising:

    a step (A) of mixing carbon dioxide and hydrogen to obtain a make-up gas;
    a step (B) of raising pressure of the make-up gas, and then mixing the make-up gas with a recycled gas recovered from an exit gas of a synthesis reactor to obtain a synthesis reactor-feeding gas;
    a step (C) of preheating the synthesis reactor-feeding gas by exchanging heat with the exit gas from the synthesis reactor as a heating source; and
    a step (D) of feeding the preheated synthesis reactor-feeding gas to the synthesis reactor and bringing into contact with a catalyst to synthesize methanol.

2. The method for producing methanol according to claim 1, further comprising a step (E) of cooling the exit gas by exchanging heat with the synthesis reactor-feeding gas as a cooling source.

3. The method for producing methanol according to claim 2, further comprising a step (F) of performing gas-liquid separation on the exit gas cooled in the step (E) to obtain a gas phase containing an unreacted gas.

4. The method for producing methanol according to claim 3, further comprising a step (G) of discharging at least a part of the gas phase obtained in the step (F) as a purge gas out of a synthesis system, recovering a part or all of the remaining gas phase as the recycled gas and mixing with the make-up gas.

5. The method for producing methanol according to claim 3, further comprising:

a step (H) of recovering at least a part of the gas phase obtained in the step (F) as a purge gas, and separating the purge gas into a first separated stream and a second separated stream;

a step (I) of discharging a part of the first separated stream out of the synthesis system as an unrecovered gas, recovering a part or all of the remaining first separated stream as a recovered gas and mixing with the make-up gas; and

a step (J) of discharging the second separated stream out of the synthesis system.

6. The method for producing methanol according to claim 2 or 3, further comprising:

a step (K) of lowering pressure of a liquid phase obtained by gas-liquid separation of the exit gas cooled in the step (E); and

a step (L) of feeding a flash gas generated during pressure lowering in the step (K) to a scrubber.

7. The method for producing methanol according to claim 6, further comprising a step (M) of reusing, as a raw material, a vent gas generated from the scrubber generated in the step (L).

8. The method for producing methanol according to claim 7, wherein in the step (M), depending on a pressure of the vent gas, it is selected either the vent gas is joined with the make-up gas or with a back stream of equipment that recovers carbon dioxide from an exhaust gas or atmosphere.

9. The method for producing methanol according to claim 6, further comprising a step (N) of joining the vent gas generated from the scrubber generated in the step (L) with the purge gas.

10. The method for producing methanol according to claim 1 or 2, wherein a content ratio between carbon dioxide and hydrogen in the make-up gas is hydrogen/carbon dioxide = 2.5 to 4.0 (volume ratio).

11. The method for producing methanol according to claim 1 or 2, wherein a circulation ratio, which is a ratio of a molar flow rate of the recycled gas to a molar flow rate of the make-up gas, is 1.0 to 7.0.

12. A methanol production apparatus for producing methanol using carbon dioxide and hydrogen as raw materials, the apparatus comprising:

a synthesis reactor;

first mixing means that mixes carbon dioxide and hydrogen to obtain a make-up gas;

a compressor that raises pressure of the make-up gas;

second mixing means that mixes the make-up gas raised in pressure with a recycled gas recovered from an exit gas of the synthesis reactor to obtain a synthesis reactor-feeding gas; and

a first heat exchanger that preheats the synthesis reactor-feeding gas by exchanging heat with the exit gas from the synthesis reactor as a heating source,

wherein the preheated synthesis reactor-feeding gas is brought into contact with a catalyst in the synthesis reactor to synthesize methanol.

13. The methanol production apparatus according to claim 12, wherein the first heat exchanger cools the exit gas by exchanging heat with the synthesis reactor-feeding gas as a cooling source.

14. The methanol production apparatus according to claim 12 or 13, further comprising a high-pressure separator for obtaining a gas phase containing an unreacted gas by gas-liquid separation of the cooled exit gas.

15. The methanol production apparatus according to claim 12 or 13, further comprising purge-gas discharging means for discharging at least a part of the gas phase as a purge gas out of the synthesis system,

wherein a part or all of the remaining gas phase after discharging of the purge gas is recovered as the recycled gas and mixed with the make-up gas by the second mixing means.

16. The methanol production apparatus according to claim 12 or 13, further comprising:

purge-gas recovering means for recovering at least a part of the gas phase as a purge gas;

separation equipment for separating the purge gas into a first separated stream and a second separated stream;

unrecovered-gas discharging means for discharging a part of the first separated stream out of the synthesis system as an unrecovered gas;

third mixing means for recovering a part or all of the remaining first separated stream as a recovered gas and mixing with the make-up gas; and

second-separated-stream discharging means for discharging the second separated stream out of the synthesis system.

EP 4 549 423 A1

Figure 1

Figure 2

17

Figure 3

Figure 4

## INTERNATIONAL SEARCH REPORT

International application No.

**PCT/JP2023/019530**

### A. CLASSIFICATION OF SUBJECT MATTER

*C07C 29/152*(2006.01)i; *C07C 31/04*(2006.01)i; *C07B 61/00*(2006.01)i
FI:    C07C29/152; C07C31/04; C07B61/00 300

According to International Patent Classification (IPC) or to both national classification and IPC

### B. FIELDS SEARCHED

Minimum documentation searched (classification system followed by classification symbols)

C07C29/00; C07C31/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| Y | JP 06-080595 A (BABCOCK-HITACHI KABUSHIKI KAISHA) 22 March 1994 (1994-03-22)<br>  claims 1-2, fig. 1 | 1-16 |
| Y | WO 2017/175760 A1 (MITSUBISHI GAS CHEMICAL CO., INC.) 12 October 2017 (2017-10-12)<br>  claim 1, paragraph [0068] | 1-16 |
| Y | WO 2016/063872 A1 (MITSUBISHI GAS CHEMICAL COMPANY, INC.) 28 April 2016 (2016-04-28)<br>  claim 1, paragraph [0060] | 1-16 |
| Y | JP 2020-063193 A (TOYO ENGINEERING CORP.) 23 April 2020 (2020-04-23)<br>  claim 1, paragraphs [0016], [0033] | 1-16 |
| A | WO 2021/186658 A1 (HITACHI, LTD.) 23 September 2021 (2021-09-23)<br>  claim 1-17 | 1-16 |
| A | WO 2011/136345 A1 (MITSUI CHEMICALS, INC.) 03 November 2011 (2011-11-03)<br>  claims 1-6 | 1-16 |

☐ Further documents are listed in the continuation of Box C.        ☑ See patent family annex.

| | |
|---|---|
| *    Special categories of cited documents: | "T"  later document published after the international filing date or priority date and not in conflict with the application but cited to understand the principle or theory underlying the invention |
| "A"  document defining the general state of the art which is not considered to be of particular relevance | |
| "E"  earlier application or patent but published on or after the international filing date | "X"  document of particular relevance; the claimed invention cannot be considered novel or cannot be considered to involve an inventive step when the document is taken alone |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | |
| "P"  document published prior to the international filing date but later than the priority date claimed | "&"  document member of the same patent family |

| Date of the actual completion of the international search | Date of mailing of the international search report |
|---|---|
| **07 August 2023** | **15 August 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/019530**

| Patent document cited in search report | | | Publication date (day/month/year) | Patent family member(s) | | | Publication date (day/month/year) |
|---|---|---|---|---|---|---|---|
| JP | 06-080595 | A | 22 March 1994 | (Family: none) | | | |
| WO | 2017/175760 | A1 | 12 October 2017 | US claim 1, paragraph [0098] EP | 2019/0152885 3441381 | A1 A1 | |
| WO | 2016/063872 | A1 | 28 April 2016 | US claim 1, paragraph [0094] EP CN | 2017/0240492 3210961 107074702 | A1 A1 A | |
| JP | 2020-063193 | A | 23 April 2020 | (Family: none) | | | |
| WO | 2021/186658 | A1 | 23 September 2021 | (Family: none) | | | |
| WO | 2011/136345 | A1 | 03 November 2011 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019163968 A
- JP 2019527691 W
- JP 51044715 A
- JP 2695663 B
- JP 6035401 A
- JP 10272361 A
- JP 2001205089 A
- JP 8299796 A
- WO 2011048976 A